# EUROPEAN PATENT APPLICATION

(11) **EP 4 266 316 A1**
(43) Date of publication of application: **25.10.2023**
(21) Application number: 20966034.9
(22) Date of filing: 18.12.2020
(51) Int. Cl.: G16C 20/10

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: KATAOKA, Masahiro, Kawasaki-shi, Kanagawa 211-8588 (JP); HAGIWARA, Minoru, Kawasaki-shi, Kanagawa 211-8588 (JP); WADA, Mitsuhito, Kawasaki-shi, Kanagawa 211-8588 (JP); MATSUMURA, Ryo, Kawasaki-shi, Kanagawa 211-8588 (JP)
(74) Representative: Hoffmann Eitle
(86) International application number: PCT/JP2020/047562
(87) International publication number: WO 2022/130648

(57) **Abstract**

An information processing program causes a computer to execute a process including executing training of a trained model based on training data defining relations between vectors corresponding to target compounds and vectors respectively corresponding to plural subcompounds included in synthetic pathways for manufacture of the target compounds and calculating vectors of plural subcompounds corresponding to a target compound to be analyzed by inputting a vector of the target compound to be analyzed into the trained model in a case where the target compound to be analyzed has been received.

## Description

### CROSS-REFERENCE TO RELATED APPLICATION(S)

### Technical Field

The present invention relates to, for example, an information processing program.

### Background Art

Natural organic compounds that occur in nature are very promising candidates in development of new drugs, but are scarce, and manufacturing various products by using these natural organic compounds as-is is difficult. Therefore, organic compounds equivalent to scarce natural organic compounds are manufactured by use of versatile conversion reactions from materials and reagents that are inexpensive and readily available. Organic compounds equivalent to natural organic compounds will be referred to as "target compounds" in the following description.

For example, in a conventional technique, a combination of plural reagents (or materials) to be subjected to a conversion reaction for manufacture of a target compound and a synthetic pathway indicating the sequence of synthesis thereof are designed by execution of a retrosynthetic analysis of a natural organic compound. The reagents are reacted in the sequence on the basis of the synthetic pathway designed by this conventional technique and the target compound is thereby synthesized and manufactured.

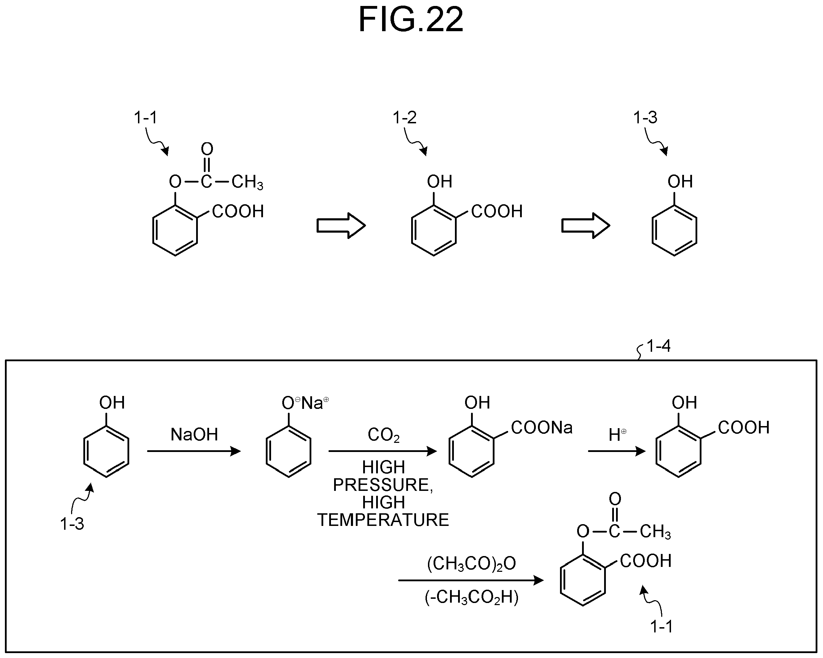

FIG. 22 is a diagram illustrating an example of retrosynthesis and a synthetic pathway. Retrosynthesis of acetylsalicylic acid 1-1 known as aspirin (an analgesic) will be described, for example. Acetylsalicylic acid 1-1 has functional groups including an ester group and a carboxyl group. Because ester is obtained from carboxylic acid and alcohol, a precursor of acetylsalicylic acid 1-1 is salicylic acid 1-2 and a reagent used is acetic anhydride. Because salicylic acid 1-2 is obtained by a Kolbe-Schmitt reaction in which carbon dioxide is reacted with a sodium salt of inexpensive phenol under high pressure, a precursor of salicylic acid is phenol 1-3. On the basis of a result of this retrosynthesis, a synthetic pathway 1-4 is designed and acetylsalicylic acid 1-1 is synthesized from phenol 1-3.

### Citation List

### Patent Literature

Patent Literature 1: Japanese Laid-open Patent Publication No. 2020-154442
Patent Literature 2: Japanese National Publication of International Patent Application No. 2001-507675

### Summary

### Technical Problem

In a case where plural reagents obtained by a retrosynthetic analysis for manufacture of a target compound are able to be replaced by other reagents having similar characteristics, synthesizing and manufacturing the target compound by changing the plural reagents to the other reagents that are readily available, more inexpensive, and are able to be subjected to a conversion reaction are effective. However, narrowing down innumerably available candidates for reagents to the replaceable reagents and determining the conversion reaction by means of this conventional technique are difficult.

In one aspect, an object of the present invention is to provide an information processing program, an information processing method, and an information processing apparatus that enable detection of reagents similar to reagents obtained by a retrosynthetic analysis of a target compound and determination of a conversion reaction therefor.

### Solution to Problem

According to an aspect of the embodiment of the invention, an information processing program causes a computer to execute a process including: executing training of a trained model based on training data defining relations between vectors corresponding to target compounds and vectors respectively corresponding to plural subcompounds included in synthetic pathways for manufacture of the target compounds and calculating vectors of plural subcompounds corresponding to a target compound to be analyzed by inputting a vector of the target compound to be analyzed into the trained model in a case where the target compound to be analyzed has been received. Advantageous Effects of Invention

Reagents similar to reagents for a target compound are able to be detected.

### BRIEF DESCRIPTION OF DRAWINGS

FIG. 1 is a diagram illustrating an example of a process in a training phase of an information processing apparatus according to a first embodiment.
FIG. 2 is a diagram illustrating an example of a process in an analysis phase of the information processing apparatus according to the first embodiment.
FIG. 3 is a functional block diagram illustrating a configuration of the information processing apparatus according to the first embodiment.
FIG. 4 is a diagram illustrating an example of a data structure of a chemical structural formula file.
FIG. 5 is a diagram illustrating an example of a group dictionary.
FIG. 6 is a diagram illustrating an example of a reagent dictionary.
FIG. 7A is a diagram illustrating an example of a subcompound dictionary.
FIG. 7B is a diagram illustrating an example of a target compound dictionary.
FIG. 7C is a diagram illustrating an example of a common structure dictionary.
FIG. 8 is a diagram illustrating an example of a data structure of a group vector table.
FIG. 9 is a diagram illustrating an example of a data structure of a reagent vector table.
FIG. 10A is a diagram illustrating an example of a data structure of a subcompound vector table.
FIG. 10B is a diagram illustrating an example of a data structure of a target compound vector table.
FIG. 10C is a diagram illustrating an example of a data structure of a common structure vector table.
FIG. 11 is a diagram illustrating an example of a data structure of a group inverted index;
FIG. 12 is a diagram illustrating an example of a data structure of a reagent inverted index;
FIG. 13A is a diagram illustrating an example of a data structure of a subcompound inverted index.
FIG. 13B is a diagram illustrating an example of a data structure of a target compound inverted index.
FIG. 13C is a diagram illustrating an example of a data structure of a common structure inverted index.
FIG. 14 is a diagram illustrating an example of a data structure of a retrosynthetic analysis table.
FIG. 15 is a first flowchart illustrating a procedure by the information processing apparatus according to the first embodiment.
FIG. 16 is a second flowchart illustrating a procedure by the information processing apparatus according to the first embodiment.
FIG. 17 is a diagram illustrating an example of a process in a training phase of an information processing apparatus according to a second embodiment.
FIG. 18 is a diagram illustrating a process by the information processing apparatus according to the second embodiment.
FIG. 19 is a functional block diagram illustrating a configuration of the information processing apparatus according to the second embodiment.
FIG. 20 is a flowchart illustrating a procedure by the information processing apparatus according to the second embodiment.
FIG. 21 is a diagram illustrating an example of a hardware configuration of a computer that implements functions that are the same as those of the information processing apparatuses according to the embodiments.
FIG. 22 is a diagram illustrating an example of retrosynthesis and a synthetic pathway.

### Description of Embodiments

Embodiments of an information processing program, an information processing method, and an information processing apparatus disclosed in the present application will hereinafter be described in detail on the basis of the drawings. The present invention is not limited by these embodiments.

### First Embodiment

An example of a process by an information processing apparatus according to a first embodiment will be described. It is assumed that the information processing apparatus according to the first embodiment executes beforehand by preprocessing: a process of calculating a vector of a target compound; and a process of calculating vectors of subcompounds (reagents) corresponding to the target compound. A synthetic pathway for manufacture of the target compound is designed by execution of a retrosynthetic analysis of the target compound, and a relation between the target compound, and the reagents and a conversion reaction for synthesis and manufacture of the target compound is determined.

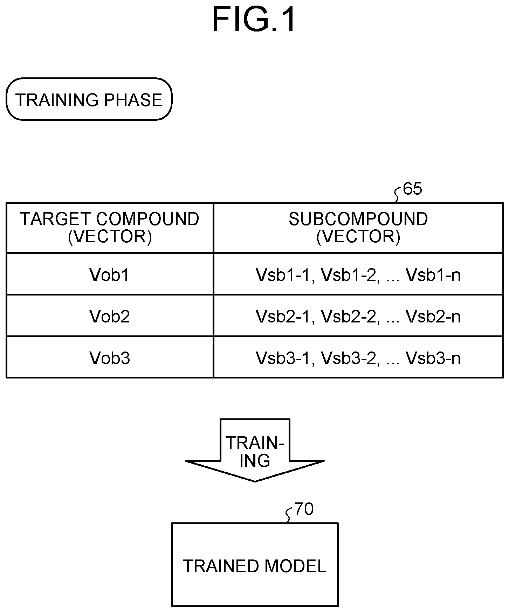

FIG. 1 is a diagram illustrating an example of a process in a training phase of the information processing apparatus according to the first embodiment. As illustrated in FIG. 1, the information processing apparatus executes training of a trained model 70 by using training data 65. The trained model 70 corresponds to, for example, a convolutional neural network (CNN) or a recurrent neural network (RNN) .

The training data 65 define relations each between: a vector of a target compound that has actually been subjected to a retrosynthetic analysis and synthesized in the past; and vectors of plural subcompounds used for a retrosynthetic analysis and synthesis of the target compound. For example, the vector of a target compound corresponds to input data, and the vectors of the plural subcompounds are correct values of output data therefor.

The information processing apparatus executes training by error back propagation, so that output upon input of the vector of a target compound into the trained model 70 approaches the vectors of the subcompounds. The information processing apparatus adjusts parameters of the trained model 70 (executes machine training) by repeatedly executing the above described process on the basis of the relations included in the training data 65, the relations each being between: the vector of a target compound; and the vectors of the plural subcompounds.

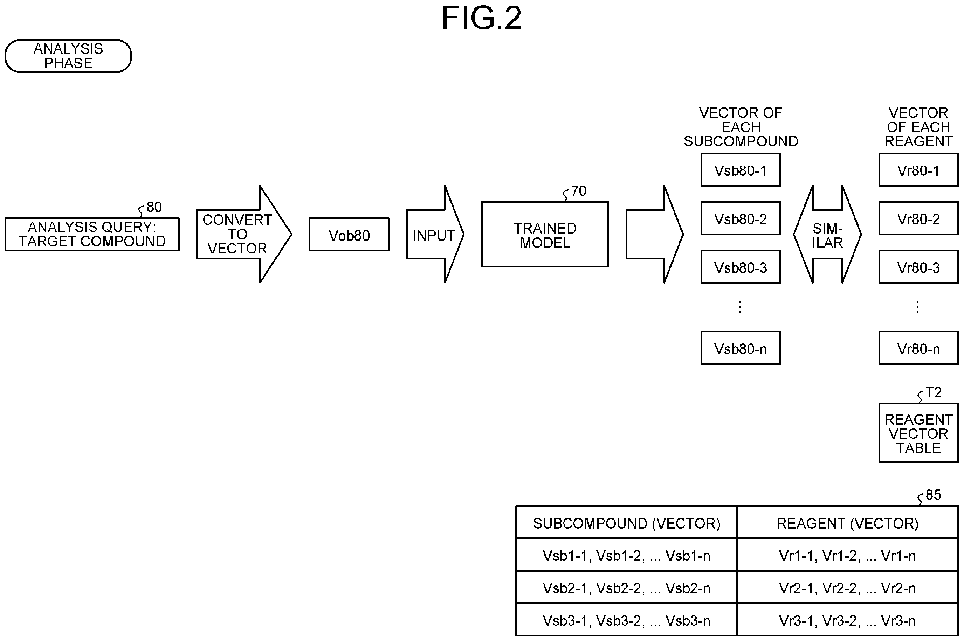

FIG. 2 is a diagram illustrating an example of a process in an analysis phase of the information processing apparatus according to the first embodiment. In the analysis phase, the information processing apparatus executes the following process by using the trained model 70 that has been trained in the training phase.

Upon receipt of an analysis query 80 that specifies a target compound, the information processing apparatus converts the target compound in the analysis query 80 to a vector Vob80. By inputting the vector Vob80 to the trained model 70, the information processing apparatus calculates plural vectors (Vsb80-1, Vsb80-2, Vsb80-3, ... Vsb80-n) corresponding to its subcompounds.

The information processing apparatus compares degrees of similarity between plural vectors (Vr80-1, Vr80-2, Vr80-3, ... Vr80-n) corresponding to reagents and stored in a reagent vector table T2 and the plural vectors (Vsb80-1, Vsb80-2, Vsb80-3, ... Vsb80-n) corresponding to the subcompounds, and makes an analysis for subcompounds and reagents similar to each other. The information processing apparatus registers vectors of the subcompounds and reagents that are similar to each other into a subcompound and reagent table 85, in association with each other.

As described above, the information processing apparatus according to the first embodiment executes training of the trained model 70 beforehand on the basis of the training data 65 that define relations between: vectors of target compounds; and vectors of subcompounds based on retrosynthetic analyses. By inputting a vector of an analysis query into the trained model 70 that has been trained, the information processing apparatus calculates vectors of subcompounds corresponding to a target compound of the analysis query. Using the vectors of the subcompounds output from the trained model 70 facilitates detection of reagents similar to the subcompounds defined by a synthetic pathway for the target compound.

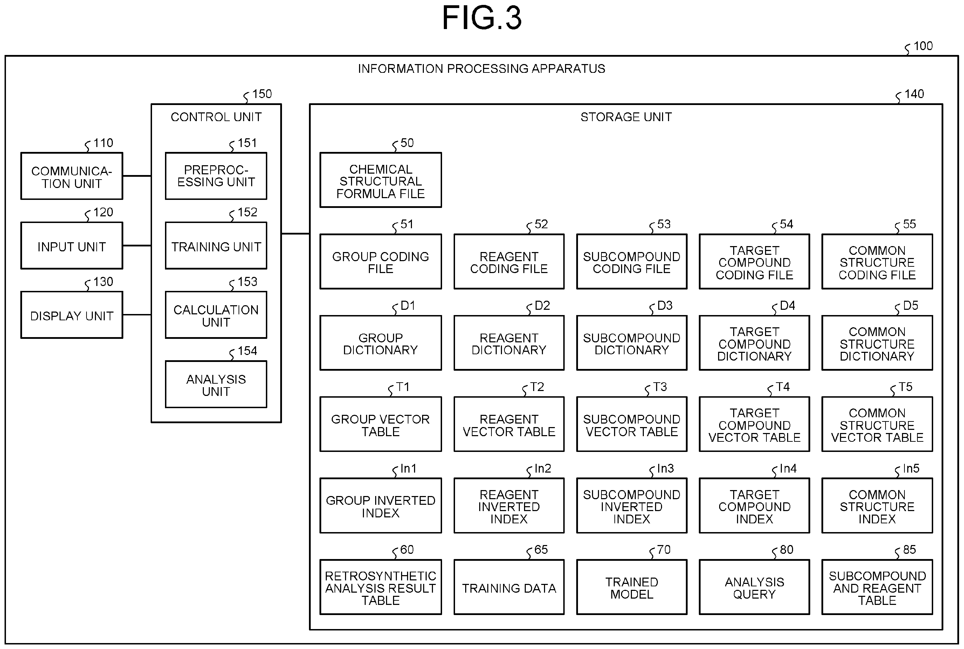

An example of a configuration of the information processing apparatus according to the first embodiment will be described next. FIG. 3 is a functional block diagram illustrating the configuration of the information processing apparatus according to the first embodiment. As illustrated in FIG. 3, this information processing apparatus 100 has a communication unit 110, an input unit 120, a display unit 130, a storage unit 140, and a control unit 150.

The communication unit 110 is connected to, for example, an external device by wire or wirelessly and transmits and receives information to and from, for example, the external device. For example, the communication unit 110 is implemented by, for example, a network interface card (NIC). The communication unit 110 may be connected to a network not illustrated in the drawings.

The input unit 120 is an input device that inputs various types of information to the information processing apparatus 100. The input unit 120 corresponds to, for example, a keyboard and a mouse, and/or a touch panel.

The display unit 130 is a display device that displays information output from the control unit 150. The display unit 130 corresponds to for example, a liquid crystal display, an organic electro luminescence display, or a touch panel.

The storage unit 140 has a chemical structural formula file 50, a group coding file 51, a reagent coding file 52, a subcompound coding file 53, a target compound coding file 54, and a common structure coding file 55. The storage unit 140 has a group dictionary D1, a reagent dictionary D2, a subcompound dictionary D3, a target compound dictionary D4, and a common structure dictionary D5. The storage unit 140 has a group vector table T1, a reagent vector table T2, a subcompound vector table T3, a target compound vector table T4, and a common structure vector table T5. The storage unit 140 has a group inverted index In1, a reagent inverted index In2, a subcompounds inverted index In3, a target compound inverted index In4, and a common structure index In5. The storage unit 140 has a retrosynthetic analysis result table 60, the training data 65, the trained model 70, the analysis query 80, and the subcompound and reagent table 85.

The storage unit 140 is implemented by, for example: a semiconductor memory element, such as a random access memory (RAM) or a flash memory; or a storage device, such as a hard disk or an optical disk.

The chemical structural formula file 50 is information including rational formulae of plural functional groups, and combining rational formulae of functional groups of smallest units forms a rational formula of a primary structure or a secondary structure. In the description of this first embodiment, for example, a rational formula of a primary structure corresponds to a "subcompound" or "reagent", and a rational formula of a secondary structure (or a higher-order structure) corresponds to a "target compound (or natural organic compound)".

For example, the chemical structural formula file 50 is divided into: a subcompound (reagent) description area where rational formulae corresponding to subcompounds (or reagents) are described; and a target compound description area where rational formulae corresponding to target compounds are described. Furthermore, the chemical structural formula file 50 may include information in the retrosynthetic analysis result table 60 described later.

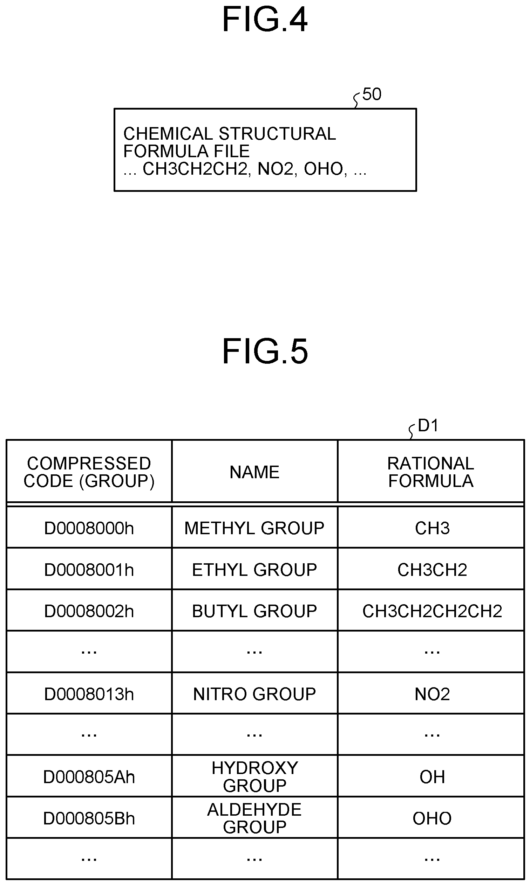

FIG. 4 is a diagram illustrating an example of a data structure of a chemical structural formula file. A rational formula (chemical structural formula) is a formula indicating the arrangement of elements composing a compound and may be described by, for example, the SMILES method.

The group coding file 51 for functional groups is a file resulting from compression of the chemical structural formula file 50 in units of groups. As described later, the group coding file 51 is generated on the basis of the chemical structural formula file 50 and the group dictionary D1.

The reagent coding file 52 is a file generated on the basis of a reagent compression area of the group coding file 51 and is a file that has been compressed in units of reagents. A compressed code of one reagent corresponds to a combination of compressed codes of plural groups. As described later, the reagent coding file 52 is generated on the basis of: the compressed codes in the reagent compression area; and the reagent dictionary D2.

The subcompound coding file 53 is a file generated on the basis of the group coding file 51 and is file that has been compressed in units of subcompounds. A compressed code of one subcompound corresponds to a combination of compressed codes of plural groups. As described later, the subcompound coding file 53 is generated on the basis of: compressed codes in a subcompound compression area; and the subcompound dictionary D3.

The target compound coding file 54 is a file generated on the basis of a target compound compression area of the group coding file 51 and is a file that has been compressed in units of target compounds. A compressed code of one target compound corresponds to a combination of compressed codes of plural groups. As described later, the target compound coding file 54 is generated on the basis of: the compressed codes in the target compound compression area; and the target compound dictionary D4.

The common structure coding file 55 is a file generated on the basis of the group coding file 51 and is a file that has been compressed in units of common structures. A compressed code of one common structure corresponds to a combination of compressed codes of plural groups. As described later, the common structure coding file 55 is generated on the basis of: compressed codes in a common structure area; and the common structure dictionary D5.

The group dictionary D1 defines compressed codes of groups and arrangements of elements composing the groups. FIG. 5 is a diagram illustrating an example of a group dictionary. As illustrated in FIG. 5, the group dictionary D1 has compressed codes, names, and rational formulae, in association with one another. The compressed codes are compressed codes that have been assigned to the groups. The names are examples of names of the corresponding groups. The rational formulae indicate arrangements serving as the rational formulae of the corresponding groups.

For example, a compressed code, "D0008000h", is assigned to a methyl group. A rational formula corresponding to the compressed code, "D0008000h", is "CH3". Herein, "h" is a sign indicating that the compressed code is hexadecimal.

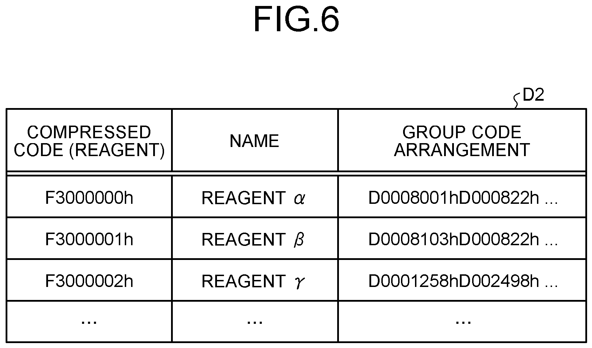

The reagent dictionary D2 defines relations each between: a compressed code of a reagent; and a combination of plural compressed codes of groups composing the reagent. FIG. 6 is a diagram illustrating an example of a reagent dictionary. As illustrated in FIG. 6, the reagent dictionary D2 has the compressed codes, names, and group code arrangements, in association with one another. The compressed codes are compressed codes that have been assigned to the reagents. The names are examples of names of the corresponding reagents. The group code arrangements are code arrangements each being a combination of plural compressed codes of groups.

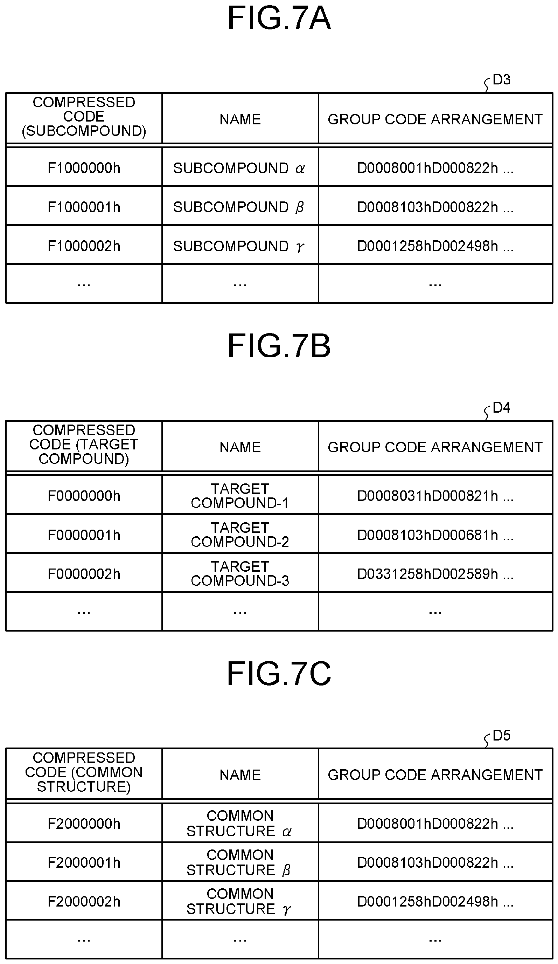

The subcompound dictionary D3 defines relations each between: a compressed code of a target compound; and a combination of plural compressed codes of groups composing the target compound. FIG. 7A is a diagram illustrating an example of a subcompound dictionary. As illustrated in FIG. 7A, the subcompound dictionary D3 has the compressed codes, names, and group code arrangements, in association with one another. The compressed codes are compressed codes that have been assigned to subcompounds. The names are examples of names of the corresponding subcompounds. The group code arrangements are code arrangements each being a combination of plural compressed codes of groups.

The target compound dictionary D4 defines relations each between: a compressed code of a target compound; and a combination of plural compressed codes of groups composing the target compound. FIG. 7B is a diagram illustrating an example of a target compound dictionary. As illustrated in FIG. 7B, the target compound dictionary D4 has the compressed codes, names, and group code arrangements, in association with one another. The compressed codes are compressed codes that have been assigned to the target compounds. The names are examples of names of the corresponding target compounds. The group code arrangements are code arrangements each being a combination of plural compressed codes of groups.

The common structure dictionary D5 corresponds to structures that are common among structures included in plural reagents. The common structure dictionary D5 defines relations each between: a compressed code of a common structure; and a combination of plural compressed codes of groups composing the common structure. FIG. 7C is a diagram illustrating an example of a common structure dictionary. As illustrated in FIG. 7C, the common structure dictionary D5 has the compressed codes, names, and group code arrangements, in association with one another. The compressed codes are compressed codes that have been assigned to the common structures. The names are examples of names of the corresponding common structures. The group code arrangements are code arrangements each being a combination of plural compressed codes of groups.

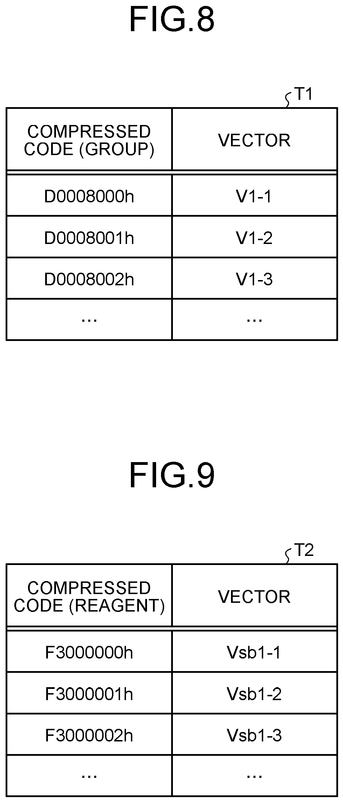

The group vector table T1 is a table defining vectors of groups. FIG. 8 is a diagram illustrating an example of a data structure of a group vector table. As illustrated in FIG. 8, this group vector table T1 has compressed codes of groups, and vectors that have been assigned to these compressed codes of the groups, in association with each other. These vectors of the groups are calculated by Poincaré embeddings.

The reagent vector table T2 is a table defining vectors of reagents. FIG. 9 is a diagram illustrating an example of a data structure of a reagent vector table. As illustrated in FIG. 9, this reagent vector table T2 has compressed codes of reagents, and vectors that have been assigned to these compressed codes of the reagents, in association with each other. The vectors of the reagents are each a result of addition of vectors of compressed codes of groups composing that reagent. The reagent vector table T2 may further hold therein characteristics, such as names of the reagents and/or rational formulae of the reagents, further in association.

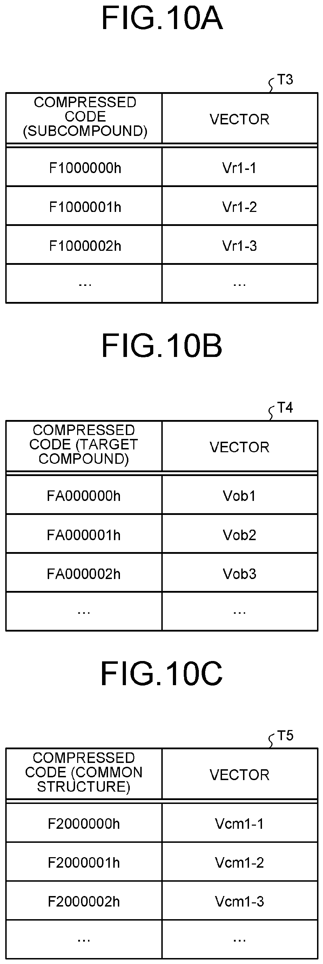

The subcompound vector table T3 is a table defining vectors of subcompounds. FIG. 8 is a diagram illustrating an example of a data structure of a subcompound vector table. As illustrated in FIG. 10A, this subcompound vector table T3 has compressed codes of subcompounds and vectors that have been assigned to these compressed codes of the subcompounds, in association with each other. The vectors of the subcompounds are each a result of addition of vectors of compressed codes of groups composing that subcompound. The subcompound vector table T3 may hold therein characteristics, such as names of the subcompounds and/or rational formulae of the subcompounds, further in association.

The target compound vector table T4 is a table defining vectors of target compounds. FIG. 10B is a diagram illustrating an example of a data structure of a target compound vector table. As illustrated in FIG. 10B, this target compound vector table T3 has compressed codes of the target compounds, and the vectors that have been assigned to the compressed codes of the target compounds, in association with each other. The vectors of the target compounds are each a result of addition of vectors of compressed codes of groups composing that target compound.

The common structure vector table T5 is a table defining vectors of common structures. FIG. 10C is a diagram illustrating an example of a data structure of a common structure vector table. As illustrated in FIG. 10C, this common structure vector table T5 has compressed codes of the common structures and vectors that have been assigned to these compressed codes of the common structures, in association with each other. The vectors of the common structures are each a result of addition of vectors of compressed codes of groups composing that common structure.

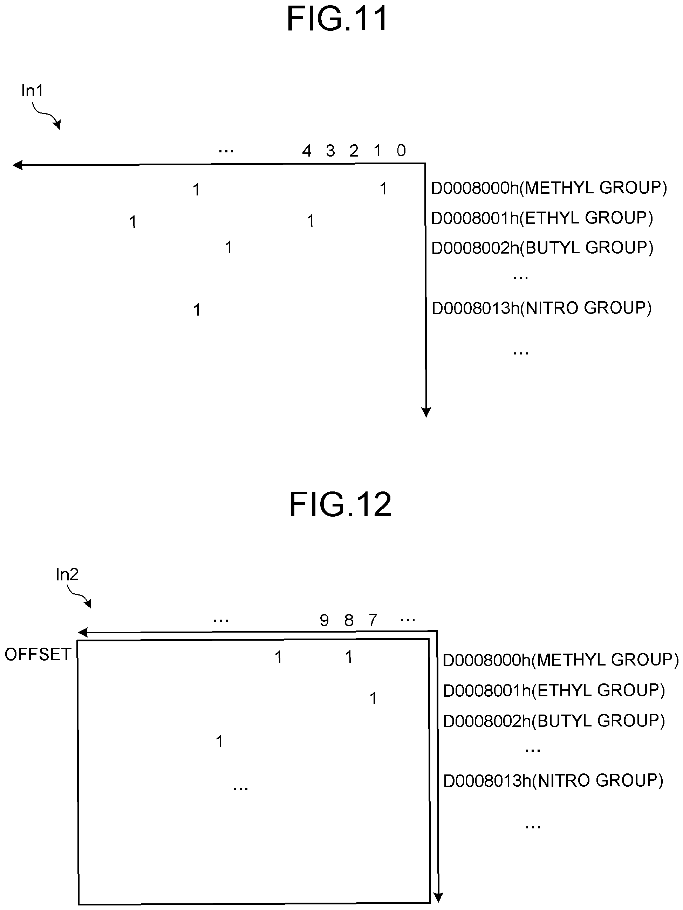

The group inverted index In1 indicates the appearance positions (offsets) in the group coding file 51 for compressed codes of groups. FIG. 11 is a diagram illustrating an example of a data structure of a group inverted index. As illustrated in FIG. 11, the horizontal axis of the group inverted index In1 is an axis corresponding to the offsets. The vertical axis of the group inverted index In1 is an axis corresponding to the compressed codes of the groups. The group inverted index In1 is represented by a bitmap of "0" or "1" and the whole bitmap is set at "0" in the initial state.

For example, it is assumed that the compressed code of the group at the head of the group coding file 51 has an offset of "0". In a case where the code, "D008000h (methyl group)", of a group is included at the second position from the head of the group coding file 51, the bit at a position where the column of the offset of "1" in the group inverted index In1 and the row of the compressed code, "D008000h (methyl group)", of the group intersect each other becomes "1".

The reagent inverted index In2 indicates the appearance positions (offsets) in the reagent coding file 52 for compressed codes of reagents. FIG. 12 is a diagram illustrating an example of a data structure of a reagent inverted index. As illustrated in FIG. 12, the horizontal axis of the reagent inverted index In2 is an axis corresponding to the offsets. The vertical axis of the reagent inverted index In2 is an axis corresponding to the compressed codes of the reagents. The reagent inverted index In2 is represented by a bitmap of "0" or "1" and the whole bitmap is set at "0" in the initial state.

For example, it is assumed that the compressed code of the reagent at the head of the reagent coding file 52 has an offset of "0". In a case where the code, "D0008000h", of a reagent is included at the ninth position from the head of the reagent coding file 52, the bit at the position where the column of the offset of "8" in the reagent inverted index In2 and the row of the compressed code, "D0008000h", of the reagent intersect each other becomes "1".

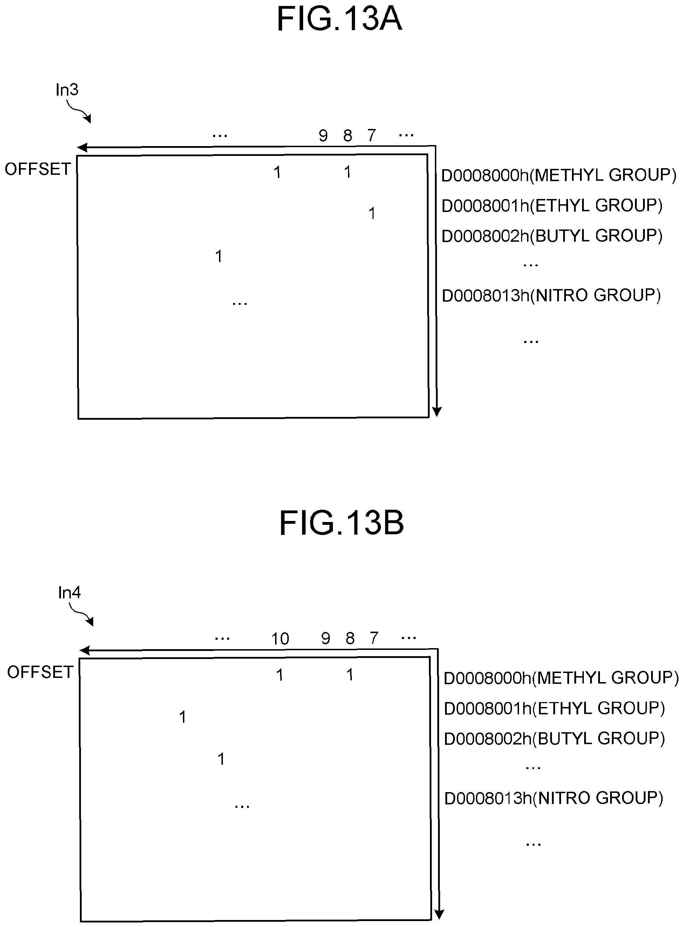

The subcompound inverted index In3 indicates the appearance positions (offsets) in the subcompound coding file 53 for compressed codes of subcompounds. FIG. 13A is a diagram illustrating an example of a data structure of a subcompound inverted index. As illustrated in FIG. 13A, the horizontal axis of the subcompounds inverted index In3 is an axis corresponding to the offsets. The vertical axis of the subcompound inverted index In3 is an axis corresponding to the compressed codes of the subcompounds. The subcompound inverted index In3 is represented by a bitmap of "0" or "1" and the whole bitmap is set at "0" in the initial state.

For example, it is assumed that the compressed code of the subcompound at the head of the subcompound coding file 53 has an offset of "0". In a case where the code, "D0008000h", of a subcompound is included at the ninth position from the head of the subcompound coding file 53, the bit at the position where the column of the offset of "8" in the subcompound inverted index In3 and the row of the compressed code, "D0008000h", of the subcompound intersect each other becomes "1".

The target compound inverted index In4 indicates the appearance positions (offsets) in the target compound coding file 54 for compressed codes of target compounds. FIG. 13B is a diagram illustrating an example of a data structure of a target compound inverted index. As illustrated in FIG. 13B, the horizontal axis of the target compound inverted index In4 is an axis corresponding to the offsets. The vertical axis of the target compound inverted index In4 is an axis corresponding to the compressed codes of the target compounds. The target compound inverted index In4 is represented by a bitmap of "0" or "1" and the whole bitmap is set at "0" in the initial state.

For example, it is assumed that the compressed code of a target compound at the head of the target compound coding file 54 has an offset of "0". In a case where the code, "D0008000h", of a target compound is included at the ninth position from the head of the target compound coding file 54, the bit at the position where the column of the offset of "8" in the target compound inverted index In4 and the row of the compressed code, "D0008000h", of the target compound intersect each other becomes "1".

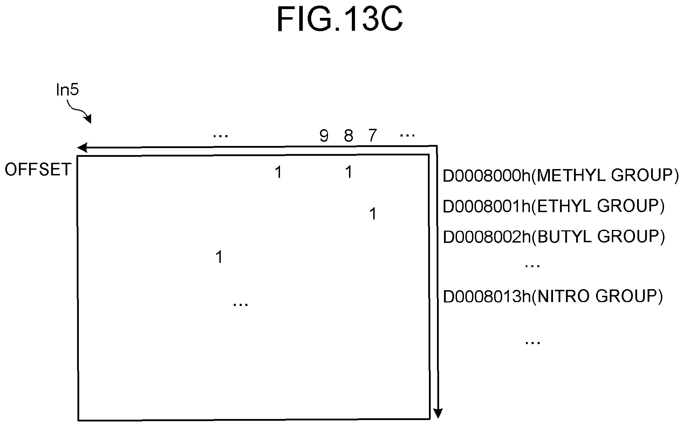

The common structure inverted index In5 indicates the appearance positions (offsets) in the common structure coding file 55 for compressed codes of common structures. FIG. 13C is a diagram illustrating an example of a data structure of a common structure inverted index. As illustrated in FIG. 13C, the horizontal axis of the common structure inverted index In5 is an axis corresponding to the offsets. The vertical axis of the common structure inverted index In5 is an axis corresponding to the compressed codes of the common structures. The common structure inverted index In5 is represented by a bitmap of "0" or "1" and the whole bitmap is set at "0" in the initial state.

For example, it is assumed that the compressed code of the common structure at the head of the common structure coding file 55 has an offset of "0". In a case where the code, "D0008000h", of a common structure is included at the ninth position from the head of the common structure coding file 55, the bit at the position where the column of the offset of "8" of the common structure inverted index In5 and the row of the compressed code, "D0008000h", of the common structure intersect each other becomes "1".

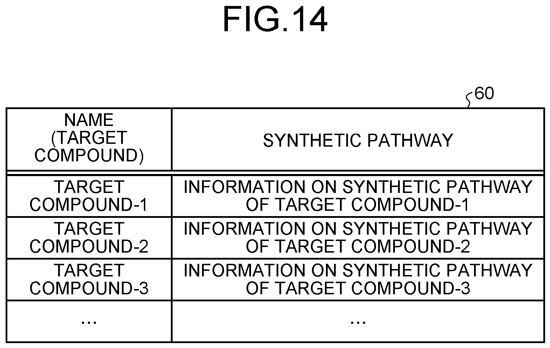

The retrosynthetic analysis result table 60 holds therein information (synthetic pathways) obtained by execution of retrosynthetic analyses for target compounds (natural organic compounds corresponding to the target compounds). FIG. 14 is a diagram illustrating an example of a data structure of a retrosynthetic analysis result table. As illustrated in FIG. 14, this retrosynthetic analysis result table 60 has names of target compounds and synthetic pathways obtained by retrosynthetic analyses for the target compounds, in association with each other. The synthetic pathways each include names of reagents reacted in that synthetic pathway.

By reference to FIG. 14, the case where the names of target compounds and the names of the subcompounds (reagents) are associated with each other has been described, but without being limited to this case, the target compounds and the names of the subcompounds (reagents) may be associated with each other by means of rational formulae. Furthermore, information in the retrosynthetic analysis result table 60 may be part of the chemical structural formula file 50.

The training data 65 define relations between vectors of target compounds and vectors of pluralities of subcompounds (reagents) used for manufacture of the target compounds. A data structure of the training data 65 corresponds to the data structure of the training data described by reference to FIG. 1.

The trained model 70 is a model corresponding to, for example, a CNN or an RNN, and parameters are set for the trained model 70.

The analysis query 80 includes information on a rational formula of a target compound to be analyzed for reagents.

The subcompound and reagent table 85 is a table holding therein vectors of subcompounds and reagents that are similar to each other, in association with each other. The subcompound and reagent table 85 has a data structure corresponding to the data structure of the subcompound and reagent table described by reference to FIG. 2.

The description of FIG. 3 will now be resumed. The control unit 150 has a preprocessing unit 151, a training unit 152, a calculation unit 153, and an analysis unit 154. The control unit 150 is implemented by, for example, a central processing unit (CPU) or a micro processing unit (MPU). Furthermore, the control unit 150 may be implemented by, for example, an integrated circuit, such as an application specific integrated circuit (ASIC) or a field programmable gate array (FPGA).

By executing various processes described below, the preprocessing unit 151 calculates, for example, a vector of a target compound and vectors of subcompounds (reagents).

For example, the preprocessing unit 151 executes a process of generating the group coding file 51, a process of generating the group vector table T1 and the group inverted index In1, and a process of generating the reagent coding file 52, the reagent vector table T2, and the reagent inverted index In2. The preprocessing unit 151 executes a process of generating the subcompound coding file 53, the subcompound vector table T3, and the subcompound inverted index In3. The preprocessing unit 151 executes a process of generating the target compound coding file 54, the target compound vector table T4, and the target compound inverted index In4. The preprocessing unit 151 executes a process of generating the training data 65.

The following description is on an example of the process in which the preprocessing unit 151 generates the group coding file 51. On the basis of the chemical structural formula file 50 and the group dictionary D1, the preprocessing unit 151 generates the group coding file 51 by repeatedly executing a process of determining a rational formula of a group included in the chemical structural formula file 50 and replacing the determined rational formula of the group with a compressed code. For example, the group coding file 51 includes a reagent compression area, a subcompound compression area, and the target compound compression area.

By executing the above described process for each rational formula included in a reagent description area of the group coding file 51, the preprocessing unit 151 generates group code arrangements for the reagent compression area. By executing the above described process for each rational formula included in a subcompound description area of the group coding file 51, the preprocessing unit 151 generates group code arrangements for the subcompound compression area. By executing the above described process for each rational formula included in a target compound description area of the group coding file 51, the preprocessing unit 151 generates group code arrangements for the target compound compression area.

The following description is on an example of the process in which the preprocessing unit 151 generates the group vector table T1 and the group inverted index In1. In generating the group vector table T1, the preprocessing unit 151 executes Poincaré embeddings.

By embedding a compressed code of a group into a Poincaré space, the preprocessing unit 151 calculates the vector of the group (the compressed code of the group). A process of calculating a vector by embedding into a Poincaré space is a technique called Poincaré embeddings. For Poincaré embeddings, for example, a technique described in Non-Patent Literature by Valentin Khrulkov1 et al., "Hyperbolic Image Embeddings", Cornell University, 2019 April 3, may be used.

Poincaré embeddings are characterized in that a vector is assigned according to the embedded position in a Poincaré space and the more similar pieces of information are to each other, the nearer the positions they are embedded at are. Therefore, groups having similar characteristics are embedded at positions that are near one another in the Poincaré space and similar vectors are thus assigned to these groups. The preprocessing unit 151 refers to a group similarity table that defines groups that are similar to one another, embeds the compressed codes of these groups into the Poincaré space, and calculates vectors of the compressed codes of these groups, although illustration thereof is omitted. The preprocessing unit 151 may execute Poincaré embeddings of the compressed codes of the groups beforehand, the compressed codes having been defined in the group dictionary D1.

By associating the groups (the compressed codes of the groups) with the vectors of the groups, the preprocessing unit 151 generates the group vector table T1. On the basis of relations between the vectors of the groups and the positions of the groups (compressed codes of the groups) in the group coding file 51, the preprocessing unit 151 generates the group inverted index In1.

The following description is on an example of the process in which the preprocessing unit 151 generates the reagent coding file 52, the reagent vector table T2, and the reagent inverted index In2. By repeatedly executing a process of replacing a group code arrangement corresponding to a reagent, with a compressed code of the reagent, on the basis of the group code arrangements in the reagent compression area included in the group coding file 51 and the reagent dictionary D2, the preprocessing unit 151 generates the reagent coding file 52.

By comparing a group code arrangement corresponding to a reagent with the group vector table T1, the preprocessing unit 151 determines a compressed code of each group included in the group code arrangement, and calculates a vector corresponding to the reagent by adding up the vectors of the determined compressed codes of the groups.

By associating the reagent (compressed code of the reagent) with the vector of the reagent, the preprocessing unit 151 generates the reagent vector table T2. On the basis of relations between the vectors of the reagents and positions of the reagents (compressed codes of the reagents) in the reagent coding file 52, the preprocessing unit 151 generates the reagent inverted index In2.

The following description is on an example of the process in which the preprocessing unit 151 generates the subcompound coding file 53, the subcompound vector table T3, and the subcompound inverted index In3. On the basis of a group code arrangement in the subcompound compression area included in the group coding file 51, and the subcompound dictionary D3, the preprocessing unit 151 generates the subcompound coding file 53 by repeatedly executing a process of replacing the group code arrangement corresponding to a subcompound, with the compressed code of the subcompound.

By comparing a group code arrangement corresponding to a subcompound, with the group vector table T1, the preprocessing unit 151 determines compressed codes of the groups included in the group code arrangement and calculates the vector corresponding to the subcompound by adding up the vectors of the determined compressed codes of the groups.

By associating subcompounds (compressed codes of the subcompounds) with vectors of the subcompounds, the preprocessing unit 151 generates the subcompound vector table T3. On the basis of relations between the vectors of the subcompounds and positions of the subcompounds (compressed codes of the subcompounds) in the subcompound coding file 53, the preprocessing unit 151 generates the subcompound inverted index In3.

The following description is on an example of the process in which the preprocessing unit 151 generates the target compound coding file 54, the target compound vector table T4, and the target compound inverted index In4. On the basis of the group code arrangements included in the target compound compression area included in the group coding file 51, and the target compound dictionary D4, the preprocessing unit 151 generates the target compound coding file 54 by repeatedly executing a process of replacing the group code arrangement corresponding to a target compound with the compressed code of the target compound.

By comparing a group code arrangement corresponding to a target compound with the group vector table T1, the preprocessing unit 151 determines compressed codes of the groups included in the group code arrangement, and calculates a vector corresponding to the target compound by adding up the vectors of the determined compressed codes of the groups.

By associating target compounds (compressed codes of the target compounds) with the vectors of the target compounds, the preprocessing unit 151 generates the target compound vector table T4. On the basis of relations between the vectors of the target compounds and positions of the target compounds (compressed codes of the target compounds) in the target compound coding file 54, the preprocessing unit 151 generates the target compound inverted index In4.

The preprocessing unit 151 may generate the common structure coding file 55, the common structure vector table T5, and the common structure inverted index In5. On the basis of the group code arrangements in the common structure area included in the group coding file 51, and the common structure dictionary D5, the preprocessing unit 151 generates the common structure coding file 55 by repeatedly executing a process of replacing the group code arrangement of a common structure with the compressed code of the common structure.

By comparing the group code arrangement corresponding to a common structure with the group vector table T1, the preprocessing unit 151 determines compressed codes of the groups included in the group code arrangement and calculates the vector corresponding to the common structure by adding up the vectors of the determined compressed codes of the groups.

By associating the common structures (compressed codes of the common structures) with the vectors of the common structures, the preprocessing unit 151 generates the common structure vector table T5. On the basis of relations between the vectors of the common structures and positions of the common structures (compressed codes of the common structures) in the common structure coding file 55, the preprocessing unit 151 generates the common structure index In5.

The following description is on an example of the process in which the preprocessing unit 151 generates the training data 65. On the basis of the retrosynthetic analysis result table 60, the preprocessing unit 151 determines a relation between the name of a target compound and names of plural subcompounds (reagents) reacted in a synthetic pathway for this target compound. On the basis of the name of the target compound and the target compound vector table T4, the preprocessing unit 151 determines the vector of the target compound. On the basis of the names of the subcompounds (reagents) and the reagent vector table T2 (or the subcompound vector table T3), the preprocessing unit 151 determines the vectors of the subcompounds (reagents). The preprocessing unit 151 determines a relation between the vector of the target compound and the vectors of the subcompounds (reagents) reacted in the synthetic pathway of the target compound and registers the determined relation into the training data 65, through this process.

The preprocessing unit 151 generates the training data 65 by repeatedly executing the above described process, for records in the retrosynthetic analysis result table 60 (names of target compounds and names of subcompounds (reagents)).

The description of FIG. 3 will now be resumed. The training unit 152 executes training of the trained model 70 by using the training data 65. A process by the training unit 152 corresponds to the process described by reference to FIG. 1. The training unit 152 obtains, from the training data 65, a pair of: a vector of a target compound; and vectors of subcompounds (reagents) corresponding to this vector of the target compound. The training unit 152 adjusts parameters of the trained model 70 by executing training by error back propagation so that the values of output from the trained model 70 in a case where the vector of the target compound has been input to the trained model 70 approaches the values of the vectors of the subcompounds (reagents).

The training unit 152 executes training of the trained model 70 by repeatedly executing the above described process for pairs of vectors of target compounds and vectors of subcompounds (reagents) in the training data 65.

In a case where the calculation unit 153 has received specification from the analysis query 80, the calculation unit 153 calculates vectors of subcompounds to be reacted through a synthetic pathway of the target compound in the analysis query 80, by using the trained model 70 that has been trained. A process by the calculation unit 153 corresponds to the process described by reference to FIG. 2. The calculation unit 153 may receive the analysis query 80 from the input unit 120 or may receive the analysis query 80 from an external device via the communication unit 110.

The calculation unit 153 obtains the rational formula of the target compound included in the analysis query 80. The calculation unit 153 compares the rational formula of the target compound with the group dictionary D1 to determines groups included in the rational formula of the target compound, and converts the rational formula of the target compound into compressed codes in units of groups.

The calculation unit 153 compares the converted compressed codes of the groups with the group vector table T1 to determine vectors of the compressed codes of the groups. By adding up the vectors of the determined compressed codes of the groups, the calculation unit 153 calculates a vector Vob80 corresponding to the target compound included in the analysis query 80.

The calculation unit 153 calculates plural vectors corresponding to the subcompounds (reagents) by inputting the vector Vob80 into the trained model 70. The calculation unit 153 outputs the calculated vectors of the subcompounds, to the analysis unit 154.

In the description hereinafter, the vectors of the subcompounds (reagents) calculated by the calculation unit 153 will each be referred to as the "analysis vector".

On the basis of the analysis vectors, the analysis unit 154 retrieves information on reagents having vectors similar to the analysis vectors. On the basis of a result of the retrieval, the analysis unit 154 registers vectors of subcompounds composing a target compound and vectors of reagents similar thereto (similar vectors described hereinafter) in association with each other, into the subcompound and reagent table 85.

For example, the analysis unit 154 calculates distances between an analysis vector and the vectors included in the reagent vector table T2 to determine any vector having a distance less than a threshold, the distance being from the analysis vector. Any vector included in the reagent vector table T2 and having a distance from the analysis vector is a "similar vector", the distance being less than the threshold.

On the basis of the reagent vector table T2, the analysis unit 154 determines the compressed code of the reagent corresponding to the similar vector, and on the basis of the determined compressed code of the reagent, the reagent dictionary D2, and the group dictionary D1, the analysis unit 154 determines the rational formula corresponding to the compressed code of the reagent. Characteristics of the reagent may also be associated in the reagent vector table T2, and in this case, the analysis unit 154 obtains the characteristics of the reagent corresponding to the similar vector. By executing this process, the analysis unit 154 retrieves the rational formula of the reagent corresponding to the similar vector and the characteristics of the reagent, and registers a result of the retrieval into the subcompound and reagent table 85.

By repeatedly executing the above described process for the analysis vectors, the analysis unit 154 may retrieve, for each of the analysis vectors, the rational formula of the reagent corresponding to the similar vector and the characteristics of the reagent, and register them into the subcompound and reagent table 85. The analysis unit 154 may output the subcompound and reagent table 85 to the display unit 130 to cause the display unit 130 to display the subcompound and reagent table 85, or may transmit the subcompound and reagent table 85 to an external device connected to a network.

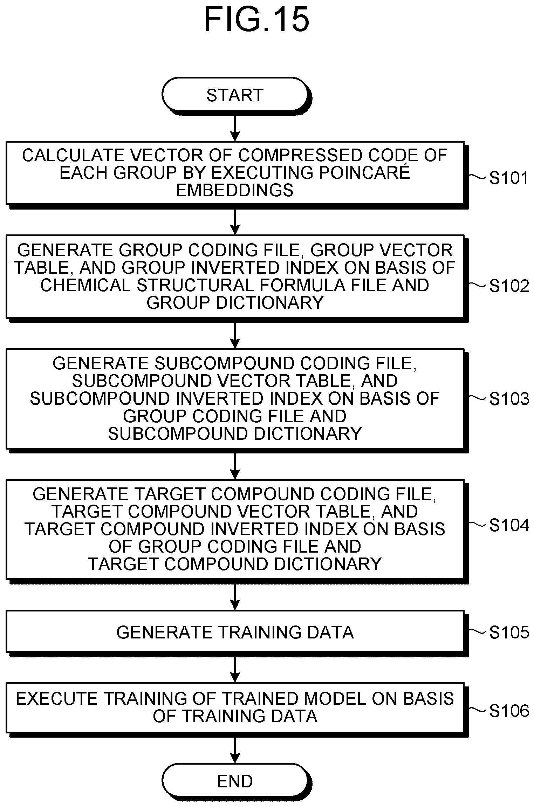

An example of a procedure by the information processing apparatus 100 according to the first embodiment will be described next. FIG. 15 is a first flowchart illustrating a procedure by the information processing apparatus according to the first embodiment. As illustrated in FIG. 15, the preprocessing unit 151 of the information processing apparatus 100 calculates vectors of compressed codes of groups by executing Poincaré embeddings (Step S101).

On the basis of the chemical structural formula file 50 and the group dictionary D1, the preprocessing unit 151 generates the group coding file 51, the group vector table T1, and the group inverted index In1 (Step S102).

On the basis of the group coding file 51 and the subcompound dictionary D3, the preprocessing unit 151 generates the subcompound coding file 53, the subcompound vector table T3, and the subcompound inverted index In3 (Step S103).

On the basis of the group coding file 51 and the target compound dictionary, the preprocessing unit 151 generates the target compound coding file 54, the target compound vector table T4, and the target compound inverted index In4 (Step S104).

On the basis of the retrosynthetic analysis result table 60, the preprocessing unit 151 determines a relation between a vector of a target compound and vectors of subcompounds (reagents) for manufacturing this target compound, to generate training data 65 (Step S105).

On the basis of the training data 65, the training unit 152 of the information processing apparatus 100 executes training of a trained model (Step S106).

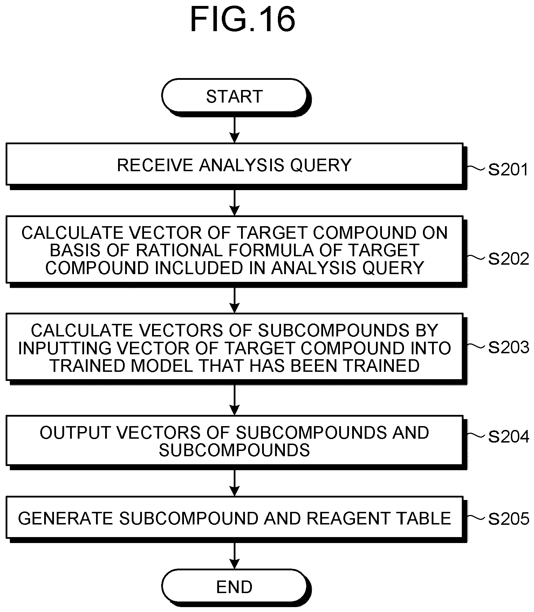

FIG. 16 is a second flowchart illustrating a procedure by the information processing apparatus according to the first embodiment. The calculation unit 153 of the information processing apparatus 100 receives the analysis query 80 (Step S201).

On the basis of the rational formula of the target compound included in the analysis query 80, the calculation unit 153 calculates the vector of the target compound (Step S202).

By inputting the calculated vector of the target compound into the trained model 70 that has been trained, the calculation unit 153 calculates vectors of its subcompounds (Step S203). The calculation unit 153 outputs the vectors of the subcompounds and the subcompounds (Step S204).

By using the vectors of the subcompounds output from the trained model 70 and the reagent vector table T2, the analysis unit 154 retrieves vectors of reagents similar to the subcompounds composing the target compound and generates the subcompound and reagent table 85 (Step S205).

Effects of the information processing apparatus 100 according to the first embodiment will be described next. In the training phase, the information processing apparatus 100 executes training of the trained model 70 beforehand, on the basis of the training data 65 defining relations between vectors of target compounds and vectors of subcompounds (reagents) based on retrosynthetic analyses. In the analysis phase, by inputting a vector of an analysis query into the trained model 70 that has been trained, the information processing apparatus 100 calculates vectors of subcompounds (reagents) corresponding to the target compound in the analysis query. Using the vectors of the subcompounds (reagents) output from the trained model 70 facilitates detection of reagents similar to the subcompounds defined in a synthetic pathway for the target compound.

A target compound that is a secondary structure of functional groups is composed of subcompounds that are each a primary structure of plural functional groups. Furthermore, transition of vectors of the plural functional groups composing a subcompound is gentle, but the vector of the functional group at the tail of a subcompound and the vector of the functional group at the head of another subcompound following that subcompound are often quite different from each other. By performing machine training on the basis of the vector of the secondary structure of the functional groups of the target compound that has actually been subjected to a retrosynthetic analysis in the past and the vectors of the primary structures of the functional groups of the subcompounds, precision of retrosynthetic analyses of organic compounds is able to be improved.

### Second Embodiment

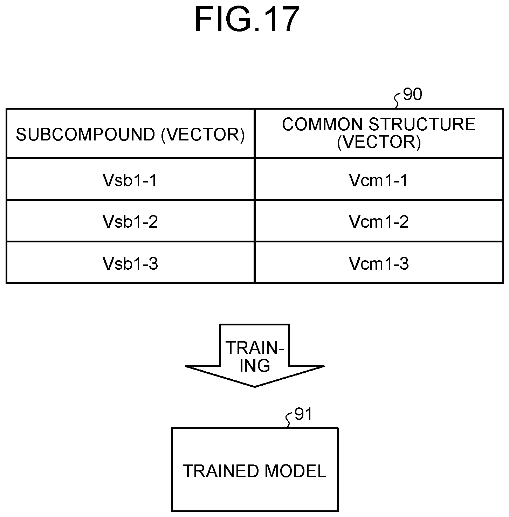

FIG. 17 is a diagram illustrating an example of a process in a training phase of an information processing apparatus according to a second embodiment. As illustrated in FIG. 17, by using training data 90, the information processing apparatus executes training of a trained model 91. The trained model 91 corresponds to, for example, a CNN or an RNN.

The training data 90 define relations between: vectors of plural subcompounds for synthesis of a target compound and vectors of common structures that are maintained in conversion reactions based on reagents. For example, vectors of subcompounds correspond to input data, and vectors of plural common structures are correct values.

The information processing apparatus executes training by error back propagation, so that output upon input of a vector of subcompound to the trained model 91 approaches the vector of each common structure. The information processing apparatus adjusts parameters of the trained model 91 (executes machine training) by repeatedly executing the above described process on the basis of the relations between: the vectors of the subcompounds included in the training data 90; and the vectors of the common structures.

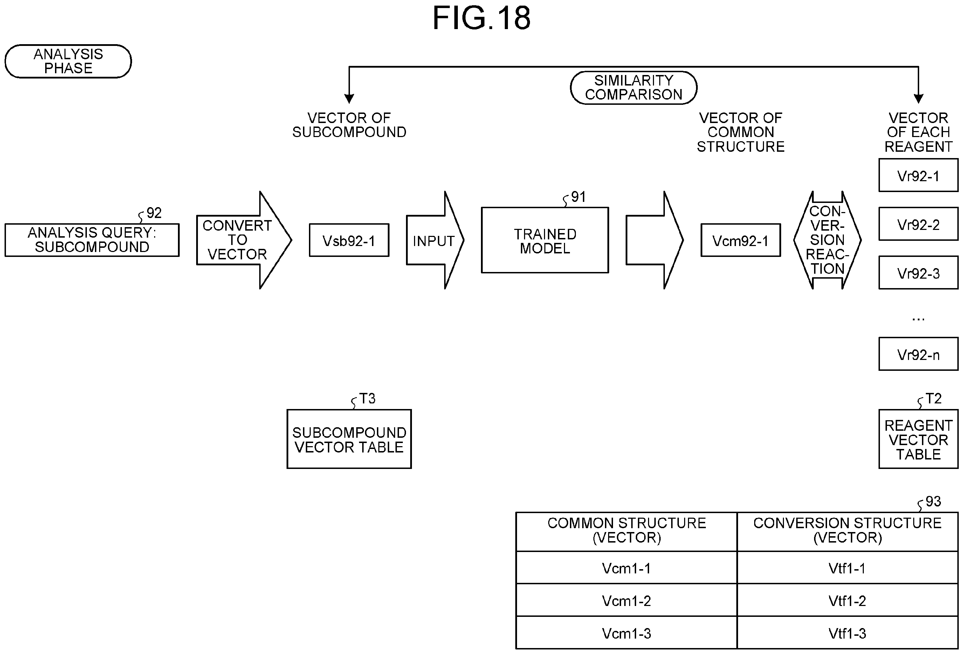

FIG. 18 is a diagram illustrating a process by the information processing apparatus according to the second embodiment. Similarly to the information processing apparatus 100 of the first embodiment, the information processing apparatus according to the second embodiment may train a trained model 70 beforehand. Furthermore, as described already by reference to FIG. 17, the information processing apparatus trains the trained model 91 that is different from the trained model 70. The trained model 70 outputs vectors of subcompounds in a case where a vector of an analysis query (target compound) 80 is input to the trained model 70. The trained model 91 outputs a vector of a common structure in a case where a vector of an analysis query (subcompound) 92 is input to the trained model 91.

Upon receipt of the analysis query 92 specifying a subcompound, the information processing apparatus converts the subcompound of the analysis query 92 into a vector Vsb92-1 by using a subcompound vector table T3. By inputting the vector Vsb92-1 of the subcompound into the trained model 91, the information processing apparatus calculates a vector Vcm92-1 corresponding to a common structure.

The information processing apparatus then compares the vector Vsb92-1 of the subcompound with vectors of plural reagents included in a reagent vector table T2. The reagent vector table T2 corresponds to the reagent vector table T2 described with reference to the first embodiment.

For the vector Vsb92-1 of the subcompound, the information processing apparatus determines a vector of a similar reagent. For example, it is assumed that the vector of the reagent similar to the vector Vsb92-1 of the subcompound is Vr92-1. A vector of a common structure common to the subcompound having the vector Vsb92-1 and the reagent having the vector Vr92-1 is then found to be the vector Vcm92-1 output from the trained model 91. Furthermore, a result of subtraction of the vector Vcm92-1 of the common structure from the vector Vr92-1 of the reagent is a vector of a difference structure (a vector of a conversion structure) corresponding to difference between the reagent and subcompound similar to each other.

The information processing apparatus registers the relation between the vector of the common structure and the vector of the conversion structure into a common structure and conversion structure table 93. By repeatedly executing the above described process for vectors of subcompounds, the information processing apparatus generates the common structure and conversion structure table 93.

By using a relation, "vector of subcompound - vector of common structure = vector of reagent - vector of common structure + vector of conversion structure", the information processing apparatus may calculate a vector of a conversion structure.

As described above, the information processing apparatus according to the second embodiment inputs the vector of the analysis query 92 into the trained model 91 that has been trained and thereby calculates the vector of each common structure corresponding to the subcompound of the analysis query. Furthermore, by subtraction of the vector of the common structure from the vector of a reagent similar to the subcompound, the vector of a conversion structure corresponding to difference between the subcompound and reagent similar to each other is calculated. Using the vectors of the common structures and vectors of the conversions structures facilitates analysis for better reagents that are usable in synthesis and manufacture of target compounds.

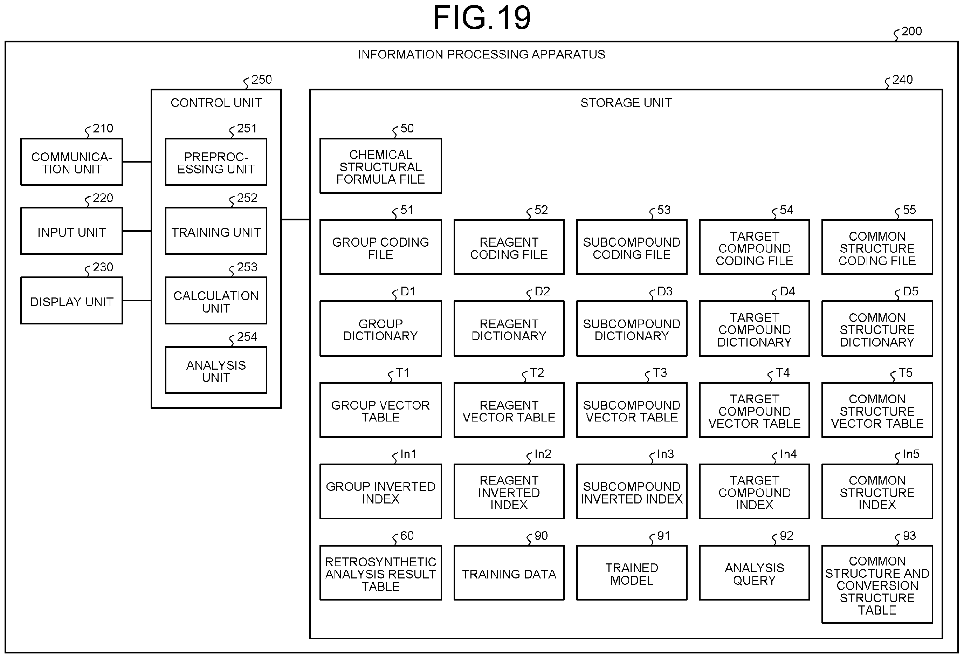

An example of a configuration of the information processing apparatus according to the second embodiment will be described next. FIG. 19 is a functional block diagram illustrating the configuration of the information processing apparatus according to the second embodiment. As illustrated in FIG. 19, this information processing apparatus 200 has a communication unit 210, an input unit 220, a display unit 230, a storage unit 240, and a control unit 250.

Description related to the communication unit 210, input unit 220, and the display unit 230 is similar to the description related to the communication unit 110, the input unit 120, and the display unit 130 described with respect to the first embodiment.

The storage unit 240 has a chemical structural formula file 50, a group coding file 51, a reagent coding file 52, a subcompound coding file 53, a target compound coding file 54, and a common structure coding file 55. The storage unit 240 has a group dictionary D1, a reagent dictionary D2, a subcompound dictionary D3, a target compound dictionary D4, and a common structure dictionary D5. The storage unit 240 has a group vector table T1, the reagent vector table T2, the subcompound vector table T3, a target compound vector table T4, and a common structure vector table T5. The storage unit 240 has a group inverted index In1, a reagent inverted index In2, a subcompound inverted index In3, a target compound index In4, and a common structure index In5. The storage unit 240 has a retrosynthetic analysis result table 60, the training data 90, the trained model 91, and the analysis query 92. The storage unit 240 has the common structure and conversion structure table 93.

The storage unit 240 is implemented by, for example: a semiconductor memory element, such as a RAM or a flash memory; or a storage device, such as a hard disk or an optical disk.

Description related to the chemical structural formula file 50, the group coding file 51, the reagent coding file 52, the subcompound coding file 53, the target compound coding file 54, and the common structure coding file 55 is similar to what has been described with respect to the first embodiment. Description related to the group dictionary D1, the reagent dictionary D2, the subcompound dictionary D3, the target compound dictionary D4, and the common structure dictionary D5 is similar to what has been described with respect to the first embodiment. Description related to the group vector table T1, the reagent vector table T2, the subcompound vector table T3, the target compound table T4, and the common structure vector table T5 is similar to what has been described with respect to the first embodiment. Description related to the group inverted index In1, the reagent inverted index In2, the subcompound inverted index In3, the target compound index In4, and the common structure index In5 is similar to what has been described with respect to the first embodiment. The retrosynthetic analysis result table 60 is similar to that described with respect to the first embodiment. The training data 90 are similar to that described by reference to FIG. 17. Description related to the trained model 91 and the analysis query 92 is similar to what has been described with reference to FIG. 18.

As described by reference to FIG. 18, the common structure and conversion structure table 93 includes information on conversion structure vectors for conversion reactions from reagents similar to common structure vectors to subcompounds. In FIG. 18, for example, the common structure and conversion structure table 93 includes a conversion structure vector corresponding to Vcm92-1. A vector resulting from addition of the vector of a common structure and the vector of the conversion structure is the vector corresponding to the vector of the reagent.

The description of FIG. 19 will now be resumed. The control unit 250 has a preprocessing unit 251, a training unit 252, a calculation unit 253, and an analysis unit 254. The control unit 250 is implemented by, for example, a CPU or an MPU. Furthermore, the control unit 250 may be implemented by, for example, an integrated circuit, such as an ASIC or FPGA.

Description related to the preprocessing unit 251 is similar to the description of the process related to the preprocessing unit 151 described with respect to the first embodiment. The group coding file 51, the reagent coding file 52, the subcompound coding file 53, the target compound coding file 54, and the common structure coding file 55 are generated by the preprocessing unit 251. The group vector table T1, the reagent vector table T2, the subcompound vector table T3, the target compound table T4, and the common structure vector table T5 are generated by the preprocessing unit 251. The group inverted index In1, the reagent inverted index In2, the subcompound inverted index In3, the target compound index In4, and the common structure index In5 are generated by the preprocessing unit 251. The preprocessing unit 251 may obtain the training data 90 from an external device or the preprocessing unit 251 may generate the training data 90.

The training unit 252 executes training of the trained model 91 by using the training data 90. A process by the training unit 252 corresponds to the process described by reference to FIG. 17. The training unit 252 obtains a pair of a vector of a subcompound and a vector of a common structure corresponding to this vector of the subcompound, from the training data 90. The training unit 252 adjusts parameters of the trained model 91 by executing training by error back propagation so that a value of output by the trained model 91 in a case where the vector of the subcompound is input to the trained model 91 approaches the value of the vector of the common structure.

In a case where the calculation unit 253 has received specification by the analysis query 92, the calculation unit 253 calculates a vector of each common structure to be subjected to a conversion reaction via a synthetic pathway for the subcompound of the analysis query 92, by using the trained model 91 that has been trained. The calculation unit 253 outputs the calculated vector of each common structure, to the analysis unit 254.

In the description hereinafter, the vectors of common structures calculated by the calculation unit 253 will each be referred to as the "common structure vector".

On the basis of the vector of the subcompound in the analysis query 92, the common structure vector, and the reagent vector table T2, the analysis unit 254 generates the common structure and change mechanism table 93. An example of a process by the analysis unit 254 will be described hereinafter.

The analysis unit 254 calculates distances between a vector of a subcompound and vectors included in the reagent vector table T2 to determine any vector having a distance less than a threshold, the distance being from the vector of the subcompound. Any vector included in the reagent vector table T2 and having a distance less than the threshold will be referred to as the "similar vector", the distance being from the vector of the subcompound.

By subtracting the common structure vector from the similar vector, the analysis unit 254 calculates the vector of the conversion structure, and determines a correspondence relation between the common structure vector and the vector of the conversion structure. The analysis unit 254 registers the common structure vector and the vector of the conversion structure into the common structure and conversion structure table 93. By repeatedly executing the above described process, an analysis unit 245 generates the common structure and conversion structure table 93. The analysis unit 245 may output the common structure and conversion structure table 93 to the display unit 230 to cause the display unit 230 to display the common structure and conversion structure table 93, or may transmit the common structure and conversion structure table 93 to an external device connected to a network.

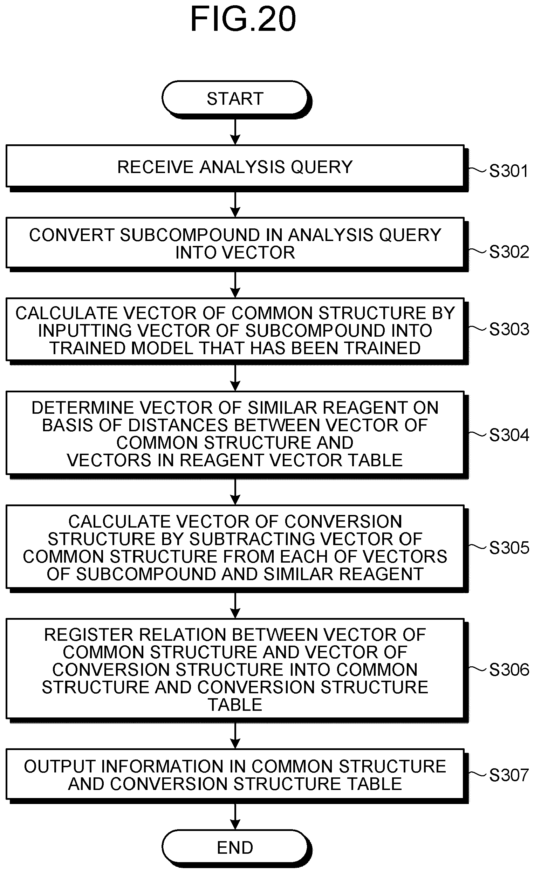

An example of a procedure by the information processing apparatus 200 according to the second embodiment will be described next. FIG. 20 is a flowchart illustrating the procedure by the information processing apparatus according to the second embodiment. The calculation unit 253 of the information processing apparatus 200 receives the analysis query 92 (Step S301).

On the basis of the subcompound vector table T3, the calculation unit 253 converts the subcompound in the analysis query 92 into a vector (Step S302).

By inputting the vector of the subcompound into the trained model 91 that has been trained, the calculation unit 253 calculates a vector of a common structure (Step S303). On the basis of distances between the vector of the common structure and vectors in the reagent vector table T2, the analysis unit 254 of the information processing apparatus 200 determines a similar reagent vector (Step S304).

The analysis unit 254 calculates a vector of a conversion structure by subtracting the vector of the common structure from each of the vectors of the subcompound and similar reagent (Step S305). The analysis unit 254 registers a relation between the vector of the common structure and the vector of the conversion structure into the common structure and conversion structure table (Step S306). The analysis unit 254 outputs information in the common structure and conversion structure table (Step S307).

Effects of the information processing apparatus 200 according to the second embodiment will be described next. The information processing apparatus 200 inputs the vector of the analysis query 92, into the trained model 91 that has been trained, and thereby calculates a vector of each common structure corresponding to the subcompound in the analysis query. Furthermore, by subtraction of the vector of each common structure from the vector of a reagent similar to the subcompound, the vector of a conversion structure corresponding to difference between the subcompound and reagent similar to each other is calculated. Using the vector of the common structure and the vector of the conversion structure facilitates analysis for better reagents that are usable in a conversion reaction into, resynthesis of, or manufacture of a target compound.

Subcompounds and reagents each have a primary structure composed of plural functional groups. Furthermore, using variance vectors of functional groups enables estimation of a functional group adjacent to a functional group and enables application to evaluation of bonding between functional groups and stability. Machine training on the basis of vectors of plural functional groups composing primary structures of subcompounds and reagents, in relation to conversion reactions from reagents to subcompounds enables improvement in precision of analysis for a conversion reaction from a reagent and resynthesis, the conversion reactions having been actually conducted in the past.

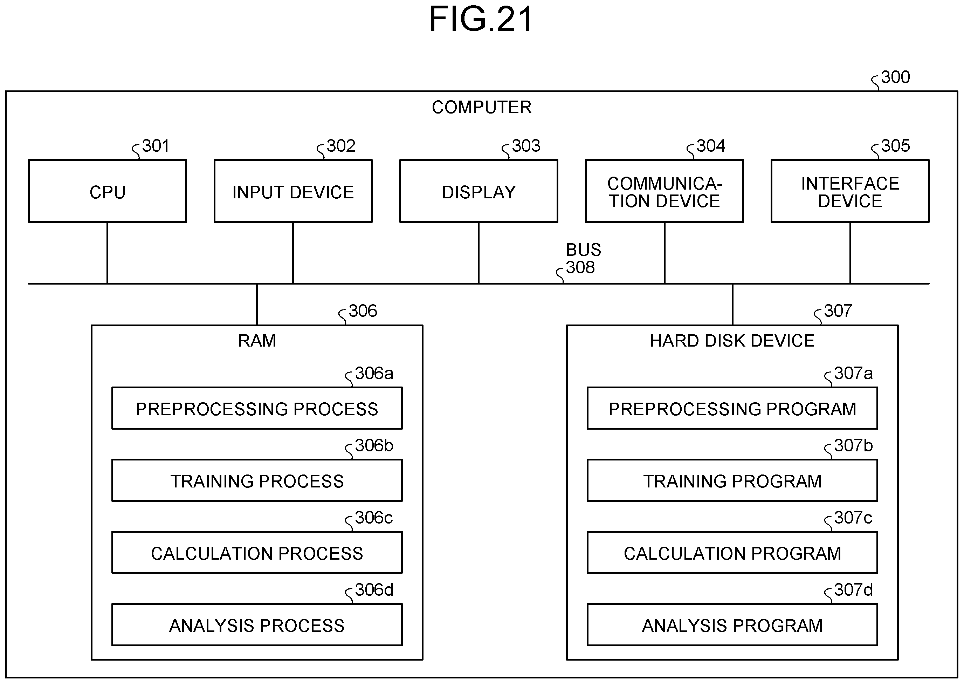

An example of a hardware configuration of a computer that implements functions that are the same as those of the above described information processing apparatus 200 (100) according to the embodiment will be described next. FIG. 21 is a diagram illustrating the example of the hardware configuration of the computer that implements functions that are the same as those of the information processing apparatus according to the embodiment.

As illustrated in FIG. 21, a computer 300 has a CPU 301 that executes various kinds of arithmetic processing, an input device 302 that receives input of data from a user, and a display 303. Furthermore, the computer 300 has: a communication device 304 that transfers data to and from, for example, an external device, via a wired or wireless network; and an interface device 305. The computer 300 also has a RAM 306 that temporarily stores therein various types of information, and a hard disk device 307. Each of these devices 301 to 307 is connected to a bus 308.

The hard disk device 307 has a preprocessing program 307a, a training program 307b, a calculation program 307c, and an analysis program 307d. Furthermore, the CPU 301 reads the programs 307a to 307d and load the read programs 307a to 307d into the RAM 306.

The preprocessing program 307a functions as a preprocessing process 306a. The training program 307b functions as a training process 306b. The calculation program 307c functions as a calculation process 306c. The analysis program 307d functions as an analysis process 306d.

A process by the preprocessing process 306a corresponds to the process by the preprocessing unit 151 or 251. A process by the training process 306b corresponds to the process by the training unit 152 or 252. A process by the calculation process 306c corresponds to the process by the calculation unit 153 or 253. A process by the analysis process 306d corresponds to the process by the analysis unit 154 or 254.

The programs 307a to 307d are not necessarily stored in the hard disk device 307 beforehand. For example, each program is stored in a "portable physical medium", such as a flexible disk (FD), a CD-ROM, a DVD, a magneto-optical disk, or an IC card, which is inserted in the computer 300. The computer 300 may then read and execute the programs 307a to 307d. Reference Signs List

- 50: CHEMICAL STRUCTURAL FORMULA FILE
- 51: GROUP CODING FILE
- 52: REAGENT CODING FILE
- 53: SUBCOMPOUND CODING FILE
- 54: TARGET COMPOUND CODING FILE
- 55: COMMON STRUCTURE CODING FILE
- 60: RETROSYNTHETIC ANALYSIS RESULT TABLE
- 65, 90: TRAINING DATA
- 70, 91: TRAINED MODEL
- 80, 92: ANALYSIS QUERY
- 85: SUBCOMPOUND AND REAGENT TABLE
- 93: COMMON STRUCTURE AND CONVERSION STRUCTURE TABLE
- 100, 200: INFORMATION PROCESSING APPARATUS
- 110, 210: COMMUNICATION UNIT
- 120, 220: INPUT UNIT
- 130, 230: DISPLAY UNIT
- 140, 240: STORAGE UNIT
- 150, 250: CONTROL UNIT
- 151, 251: PREPROCESSING UNIT
- 152, 252: TRAINING UNIT
- 153, 253: CALCULATION UNIT
- 154, 254: ANALYSIS UNIT

## Claims

1. An information processing program that causes a computer to execute a process comprising:
executing training of a trained model based on training data defining relations between vectors corresponding to target compounds and vectors respectively corresponding to plural subcompounds included in synthetic pathways for manufacture of the target compounds; and
calculating vectors of plural subcompounds corresponding to a target compound to be analyzed by inputting a vector of the target compound to be analyzed into the trained model in a case where the target compound to be analyzed has been received.

2. The information processing program according to claim 1, wherein the process further includes analyzing, based on degrees of similarity between the vectors of the plural subcompounds and vectors of plural reagents serving as replacement candidates, a reagent that is able to replace a subcompound of the target compound to be analyzed, the vectors of the plural subcompounds having been calculated by the calculating.

3. The information processing program according to claim 2, wherein the process further includes retrieving information on a rational formula of the reagent as information on the replaceable reagent and outputting a result of the retrieval.

4. The information processing program according to claim 1, wherein the target compound to be analyzed is indicated by information that is a combination of plural groups, and the process further includes calculating the vector of the target compound to be analyzed by adding up vectors of the plural groups.

5. An information processing program that causes a computer to execute a process comprising:
executing training of a trained model based on training data defining relations between vectors of plural subcompounds included in a synthetic pathway for manufacture of a target compound and vectors of common structures representing structures common to structures of the subcompounds and structures of reagents; and
calculating a vector of a common structure corresponding to a subcompound to be analyzed by inputting a vector of the subcompound to be analyzed into the trained model in a case where the subcompound to be analyzed has been received.

6. The information processing program according to claim 5, wherein the process further includes retrieving, based on similarity between the vector of the subcompound and vectors of plural reagents serving as replacement candidates, a vector of a reagent similar to the vector of the subcompound, and calculating, based on the retrieved vector of the reagent and the calculated vector of the common structure, a vector of a conversion structure representing a structure of a portion corresponding to difference between a structure of the subcompound and a structure of the retrieved reagent.

7. An information processing method comprising:
executing training of a trained model based on training data defining relations between a vector corresponding to a target compound and vectors respectively corresponding to plural subcompounds included in a synthetic pathway for manufacture of the target compound; and
calculating vectors of plural subcompounds corresponding to a target compound to be analyzed by inputting a vector of the target compound to be analyzed into the trained model in a case where the target compound to be analyzed has been received.

8. The information processing method according to claim 7, wherein the method further includes analyzing, based on degrees of similarity between the vectors of the plural subcompounds and vectors of plural reagents serving as replacement candidates, a reagent that is able to replace a subcompound of the target compound to be retrieved, the vectors of the plural subcompounds having been calculated by the calculating.

9. The information processing method according to claim 8, wherein the method further includes retrieving information on a rational formula of the reagent as information on the replaceable reagent and outputting a result of the retrieval.

10. The information processing method according to claim 9, wherein the target compound to be analyzed is indicated by information that is a combination of plural groups and the method further includes calculating the vector of the target compound to be analyzed by adding up vectors of the plural groups.

11. An information processing method comprising:
executing training of a trained model based on training data defining relations between vectors of plural subcompounds included in a synthetic pathway for manufacture of a target compound and vectors of common structures representing structures common to structures of the subcompounds and structures of reagents; and
calculating a vector of a common structure corresponding to a subcompound to be analyzed by inputting a vector of the subcompound to be analyzed into the trained model in a case where the subcompound to be analyzed has been received.

12. The information processing method according to claim 11, wherein the method further includes retrieving, based on similarity between the vector of the subcompound and vectors of plural reagents serving as replacement candidates, a vector of a reagent similar to the vector of the subcompound, and calculating, based on the retrieved vector of the reagent and the calculated vector of the common structure, a vector of a conversion structure representing a structure of a portion corresponding to difference between a structure of the subcompound and a structure of the retrieved reagent.

13. An information processing apparatus, comprising:
a training unit configured to execute training of a trained model based on training data defining relations between a vector corresponding to a target compound and vectors respectively corresponding to plural subcompounds included in a synthetic pathway for manufacture of the target compound; and
a calculation configured to calculate vectors of plural subcompounds corresponding to a target compound to be analyzed by inputting a vector of the target compound to be analyzed into the trained model in a case where the target compound to be analyzed has been received.

14. The information processing apparatus according to claim 13, further including an analysis unit configured to retrieve, based on degrees of similarity between the vectors of the plural subcompounds and vectors of plural reagents serving as replacement candidates, a reagent that is able to replace a subcompound of the target compound to be analyzed, the vectors of the plural subcompounds having been calculated by the calculation unit.

15. The information processing apparatus according to claim 14, wherein the analyzing unit is configured to retrieve information on a rational formula of the reagent as information on the replaceable reagent and outputs a result of the retrieval.

16. The information processing apparatus according to claim 13, wherein a target compound to be retrieved is represented by information that is a combination of plural groups, and the calculation unit is further configured to execute a process including calculating a vector of the target compound to be retrieved by adding up vectors of the plural groups.

17. An information processing apparatus, comprising:
a training unit configured to execute training of a trained model based on training data defining relations between vectors of plural subcompounds included in a synthetic pathway for manufacture of a target compound and vectors of common structures representing structures common to structures of the subcompounds and structures of reagents; and
a calculation unit configured to calculate a vector of a common structure corresponding to a subcompound to be analyzed by inputting a vector of the subcompound to be analyzed into the trained model in a case where the subcompound to be analyzed has been received.

18. The information processing apparatus according to claim 17, further including an analysis unit configured to retrieve, based on similarity between the vector of the subcompound and vectors of plural reagents serving as replacement candidates, a vector of a reagent similar to the vector of the subcompound, and calculates, based on the retrieved vector of the reagent and the calculated vector of the common structure, a vector of a conversion structure representing a structure of a portion corresponding to difference between a structure of the subcompound and a structure of the retrieved reagent.
